(19) Europäisches Patentamt

European Patent Office

Office européen des brevets

(11) Veröffentlichungsnummer : **0 207 106**
**B1**

(12) **EUROPÄISCHE PATENTSCHRIFT**

(45) Veröffentlichungstag der Patentschrift :
**19.07.89**

(21) Anmeldenummer : **86900060.4**

(22) Anmeldetag : **14.12.85**

(86) Internationale Anmeldenummer :
**PCT/DE 85/00526**

(87) Internationale Veröffentlichungsnummer :
**WO/8603847 (03.07.86 Gazette 86/14)**

(51) Int. Cl.⁴ : **G 02 B 21/00, G 02 B 21/24**

(54) **DURCHLICHT- UND/ODER AUFLICHTMIKROSKOP.**

(30) Priorität : **22.12.84 DE 3447128**

(43) Veröffentlichungstag der Anmeldung :
**07.01.87 Patentblatt 87/02**

(45) Bekanntmachung des Hinweises auf die Patenterteilung : **19.07.89 Patentblatt 89/29**

(84) Benannte Vertragsstaaten :
**AT CH DE FR GB LI NL**

(56) Entgegenhaltungen :
**CH–A– 396 445**
**FR–A– 551 673**
**GB–A– 6 087**
**US–A– 1 973 066**
**US–A– 2 128 394**
**US–A– 4 128 944**
**US–A– 4 277 133**

(73) Patentinhaber : **Wild Leitz GmbH**
**Ernst-Leitz-Strasse 30 Postfach 20 20**
**D-6330 Wetzlar 1 (DE)**

(72) Erfinder : **WÜRFEL, Volker**
**Hofstatt 7**
**D-6333 Braunfels-Bonbaden (DE)**

## Beschreibung

Die Erfindung betrifft ein Mikroskop für Durchlicht-, Auflicht- oder kombinierte Durchlicht/Auflicht-Beleuchtung zur Untersuchung großflächiger Objekte, wie Masken oder Wafer.

Als optische Geräte, die etwa der Inspektion bzw. der Vermessung von Wafern oder dem Ausrichten von Maske und Wafer dienen, sind Halbleitermikroskope und spezielle Maskenvergleichsmikroskope (Doppelmikroskope) bekannt, die in herkömmlicher Weise eine (oder mehrere) optische Achse(n) in Vertikalstellung und einen (oder mehrere) Objekttisch(e) in Horizontalstellung aufweisen.

Mit diesen bekannten mikroskopischen Untersuchungsgeräten gelingt es zwar, die hochempfindlichen Präzisionsobjekte (Wafer) mit ihren derzeit bekannten Abmessungen (bis 150 mm Durchmesser) in rationeller Weise zu inspizieren bzw. vermessen.

Die zukünftige Wafer-Generation wird jedoch u. a. gekennzeichnet sein durch eine Vergrößerung der Wafer-Durchmesser auf 10 Zoll und mehr sowie durch eine Zunahme der objektspezifischen Strukturdichte.

Daraus ergeben sich folgende Nachteile und Risiken bei der Wafer-Handhabung und -Untersuchung mit den bekannten Halbleitermikroskop-Typen :

— die Lage des großflächigen Wafers auf dem Horizontal-Objekttisch führt zu einer stark vergrößerten Bruch- bzw. Verkratzungsgefahr insbesondere in dessen Peripherbereichen ;

— die Bedienmöglichkeiten werden für den Beobachter in antiergonomischer Weise behindert ;

— der notwendigerweise vergrößerte Verfahrbereich des Objektkreuztisches in der horizontalen x-y-Ebene wird durch die Mikroskopierposition des Beobachters und die gerätekonstruktionsbedingten Merkmale begrenzt bzw. eingeschränkt ;

— die Kontaminationsgefahr der Wafer-Oberfläche erhöht sich in dramatischer und unkontrollierbarer Weise, was beispielsweise allein aus der Tatsache erhellt, daß bereits kleinste vom Körper bzw. von der Kleidung des Menschen abgegebene Staubpartikel, die nur mikroskopisch erfaßbar sind, die Funktionen eines hochintegrierten Schaltkreises beeinträchtigen ;

— der Flächen-Platzbedarf eines für derartige Objekte einzurichtenden, bekannten Halbleitermikroskops bzw. einer gesamten Inspektionsstation erhöht sich entsprechend ;

— für das Mikroskop ergeben sich · konstruktionsbedingte Stabilitätsprobleme.

Aus der CH-PS 396 445 ist darüber hinaus ein Durchlicht-Mikroskop zur Beobachtung von Schwebekörpern bekannt, das aus einem Mikroskop-Stativ in offener Rahmenbauweise besteht und eine vertikale Stativsäule mit aufgesetztem, horizontalen Objektivtubus sowie eine vertikale Hohlsäule mit angesetzter Beleuchtungseinrichtung und mit einem angeflanschten, vertikalen Objekttisch aufweist. Auf dem Objekttisch ist eine Flüssigkeitsküvette befestigt, in der beispielsweise kolloidale Schwebeteilchen eine Vertikalbewegung ausführen können. Der Objektraum ist bei dieser bekannten Einrichtung für Manipulierzwecke manuell zugänglich zu halten. So sind bei Verwendung einer Durchfluß-Küvette Schlauch-Anschluß-Verbindungen mit entsprechenden Rohrleitungen an der Seitenwand der Hohlsäule herzustellen. Darüber hinaus kontaktiert ein elastischer Lichtschutzring, der auf dem objektseitigen Ende des Objektivs angeordnet ist, die als Objektraum dienende Küvette. Eine flexible Strom-Zuleitung verbindet einen Steckkontakt auf der Oberseite der Hohlsäule mit der Küvette.

Dieses bekannte Mikroskop eignet sich nicht für die Beobachtung kontaminationsgefährdeter, flächiger Großobjekte, bei denen gerade ein Manipulieren in Objektnähe — also etwa ein Berühren des Objektes mittels eines Lichtschutzringes oder ein manuelles Ankoppeln einer elektrischen Verbindungsleitung an das bruchgefährdete Objekt — absolut ausgeschlossen sein muß. Vor allem aber ist diesem bekannten Mikroskop nicht die Anregung zu entnehmen, das Objekt mit einer laminaren Luft- oder Schutzgasströmung zu umspülen.

Es ist die Aufgabe der vorliegenden Erfindung, ein insbesondere für die Untersuchung großflächig-zweidimensionaler Objekte eingerichtetes Auflicht-, Durchlicht- oder Auflicht- und Durchlichtmikroskop anzugeben, das dem in Mikroskopierposition befindlichen Beobachter ergonomisch optimale Bedienmanipulationen am Gerät gestattet, das darüber hinaus ein kontaminationsfreies Inspizieren und Transportieren des Objektes erlaubt und das schließlich in stabiler, platzsparender Bauweise selbst für extrem große Objektflächen eingerichtet bzw. als integraler Baustein innerhalb einer modernen Wafer-Fertigungs-Straße verwendet werden kann.

Diese Aufgabe wird bei einem Mikroskop der eingangs genannten Art durch die Merkmale des Anspruchs 1 gelöst. Weitere Ausgestaltungen der vorliegenden Erfindung sind in den Patentansprüchen 2 bis 26 beschrieben.

In den Zeichnungen werden Ausführungsbeispiele der vorliegenden Erfindung schematisch dargestellt.

Es zeigen :

Fig. 1a : eine Seitenansicht eines in offener Vertikal-Rahmenstativ-Bauweise erstellten Vertikaltisch-Mikroskops ;

Fig. 1b : die Draufsicht des in Fig. 1a dargestellten Mikroskops ;

Fig. 1c : die Vorderansicht des in den Fig. 1a und 1b dargestellten Mikroskops ;

Fig. 2 : eine Seitenansicht eines in geschlossener Vertikal-Rahmenstativ-Bauweise mit rechteckigem Längsschnitt erstellten Vertikaltisch-Mikroskops mit einer Darstellung von einigen der in den Teilstrahlengängen vorhandenen optischen, mechanischen und elektronischen Bauglieder bzw. Module ;

Fig. 3-6 : stark schematisierte Seitenansichten von geschlossenen Vertikal-Rahmenstativ-Konstruktionen unterschiedlicher Rahmen-Längsschnittgeometrie und Objekttischebenen-Lage ;

Fig. 7 : eine stark schematisierte erste Spezialvariante mit rotatorisch gehaltertem geschlossenen Rahmenstativ in Seitenansicht ;

Fig. 8 : eine stark schematisierte zweite Spezialvariante eines Vertikal-Rahmenstativs in Seitenansicht in geschlossener Bauweise mit ortsfester Objekttischanlenkung und mit Translationsmöglichkeit des gesamten Rahmens ;

Fig. 9-13 : stark schematisierte Seitenansichten von offenen Vertikal-Rahmenstativ-Konstruktionen unterschiedlicher Rahmen-Längsschnittgeometrie, unterschiedlicher Lage der jeweiligen Objekttischebene sowie unterschiedlicher Lage der Rahmen-« Öffnung » ;

Fig. 14-16 : stark schematisierte Draufsichten von vertikalen Stativ- bzw. Einzelwand-Kombinationen ;

Fig. 17 : schematische Darstellung einer geschlossenen Rahmenstativ-Variante gemäß Fig. 6, jedoch mit extremer Quererstreckung zur optischen Achse nach Art eines Hohlprofil-Tunnels.

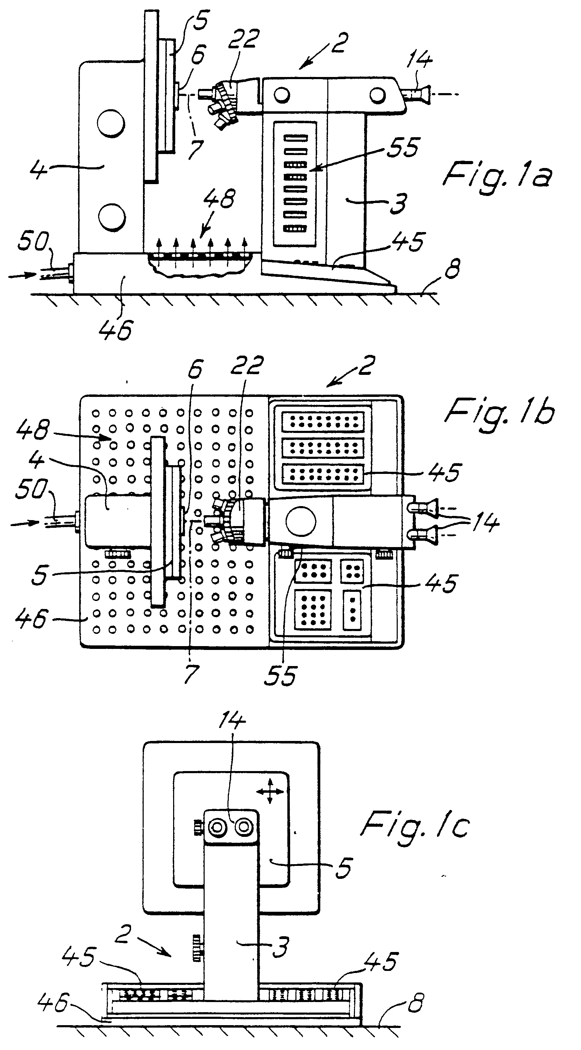

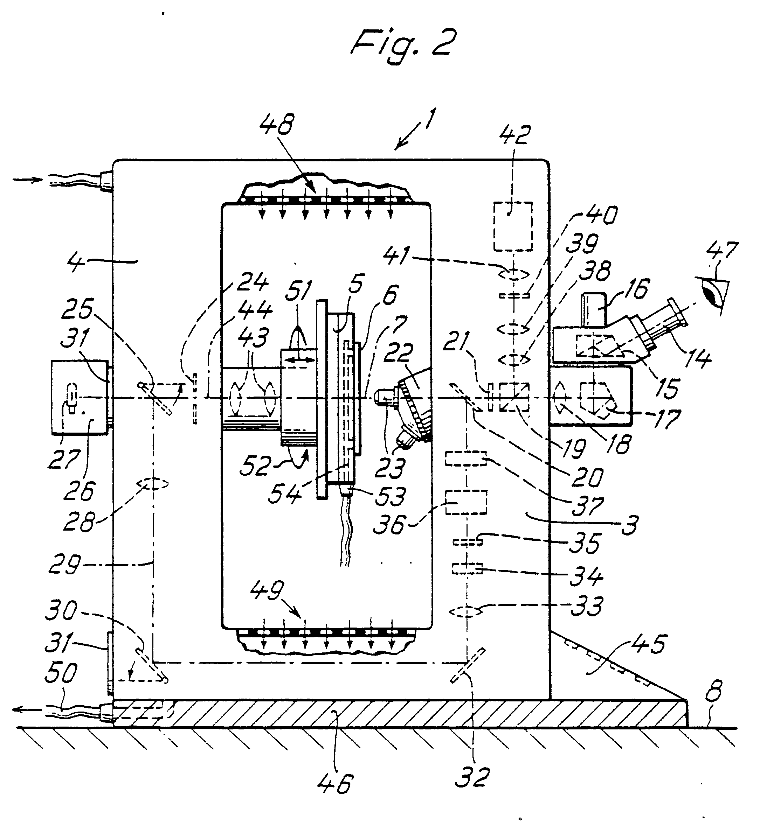

In den Fig. 1a-1c ist eine erste Ausführungsform der vorliegenden Erfindung dargestellt. Das Mikroskop-Gehäuse besteht aus einem vorderen vertikalen Stativteil 3, einem hinteren vertikalen Stativteil 4, die beide auf einer gemeinsamen Fußplatte 46 fest montiert oder in einstückiger Bauweise ausgeführt sind. Die beiden Stativteile 3 und 4 sowie die Fußplatte 46 bilden zusammen ein « offenes » Rahmenstativ 2, im Gegensatz zum in Fig. 2 dargestellten « geschlossenen » Rahmenstativ 1. Das offene Rahmenstativ 2 ist in Vertikalbauweise ausgeführt, d. h. dessen Längsschnitt-Ebene liegt in der Zeichenebene und steht senkrecht auf einer horizontalen Auflagefläche 8. Der vordere Stativteil 3 trägt eine Beobachtungseinheit, die ein Binokular 14 und einen Objektivrevolver 22 enthält ; der hintere Stativteil 4 trägt einen Objekttisch 5, beispielsweise einen Drehkreuztisch, zur Aufnahme eines Objektes 6. Im dargestellten Fall verläuft die optische Achse des Mikroskops waagerecht und die Objekttischebene steht senkrecht auf der Fläche 8. Da nachfolgend auch Ausführungsbeispiele mit von der Horizontalen abweichenden optischen Achs-Abschnitten beschrieben werden, sei bereits an dieser Stelle vermerkt, daß es nachfolgend lediglich auf die Lage-Charakterisierung desjenigen Teilbereichs der optischen Achse des Mikroskops ankommt, der vom in Wirkstellung befindlichen Objektiv des Objektivrevolvers 22 bis zum Objekt 6 bzw. bis zum Objekttisch 5 verläuft.

Wenngleich im dargestellten Fall eine Auflichtanordnung gezeigt wird, so können gleichwohl im hinteren Stativteil 4 an sich bekannte Beleuchtungseinrichtungen (Lichtquelle, Kondensoroptiken, Blenden) zur Realisierung eines kombinierten Auflicht/Durchlicht-Beleuchtungsstrahlengangs vorhanden sein.

Ebenso wurde der Einfachheit halder darauf verzichtet, Ansatzflächen für externe Lampenhäuser oder andere in der Mikroskopiertechnik gebräuchliche Module für Spezialzwecke einzuzeichnen. Ein wichtiges gerätebauliches Merkmal ist die im Seitenbereich des vorderen Stativteils 3 angeordnete Bedienleiste 55, von der aus eine Vielzahl der möglichen Bedienfunktionen auslösbar sind. Natürlich kann diese Bedienleiste auch direkt an der zum Beobachter weisenden Vorderseite des Stativteils 3 angeordnet sein.

Weitere Bedienfunktionen können vom Bedienpult 45 ausgelöst werden. Es sei hervorgehoben, daß seitens des Beobachters keinerlei Manipulationen im Nahbereich des Objektes vorgenommen werden müssen. Die Objektivwechslung, die Kreuztischverstellung, die Fokussierung, der Wechsel der Beleuchtungsart, das Anflanschen von externen bzw. das Austauschen von inkorporierten Modulen, ja selbst die Objektwechselung können automatisch erfolgen. Durch die Vertikalstellung des Objekttisches ist darüber hinaus die Gefahr einer Ablagerung von objektverschmutzenden Schwebeteilchen aus der Raumluft stark eingeschränkt. Als zusätzliche Maßnahme zur Fernhaltung von Staubpartikeln ist ein permanenter « Laminar-Flow » in Querrichtung zum flächigen Objekt vorgesehen, wie es durch die Luftzuführöffnung 50 und die im Oberbereich der Fußplatte 46 vorhandenen Luftdurchtrittsöffnungen 48, vgl. deren matrixförmige Anordnung in Fig. 1b, angezeigt ist. Es ist ebenso möglich, die Durchströmungsvorrichtung umzukehren, also eine Durchsaugevorrichtung zu realisieren.

Wenngleich eine exakte Vertikalstellung der Objekttischebene und damit des Objektes 6, beispielsweise eines Silizium-Wafers von 200 mm Durchmesser, einer permanent drohenden Staubpartikel-Berieselung die geringstmögliche Auflagefläche bietet und daher zu favorisieren ist, ist es auch möglich, den Objekttisch 5 um einen gewissen Winkelbetrag $\alpha$ in eine Schräglage nach Art eines Notenständer-Pultes zu kippen. Der Winkel $\alpha$ sollte dabei den Bereich $0° \leq \alpha \leq 30°$ nicht überschreiten. Dabei entspricht eine Verkippung des Objekttisches aus der Vertikallage um einen Winkel $\alpha_1$ einer Verkippung des objektseitigen Teilbereichs 7 der optischen Achse des Mikroskops aus der Horizontallage um eben diesen Winkel $\alpha_1$, da der objektseitige Teilbereich 7 immer senkrecht auf dem Objekt 6 stehen sollte. Die dafür vorgesehenen Mittel sind in den Fig. 1a-1c wie auch in den meisten der weiter unten noch zu erläuternden Figuren, nicht mit dargestellt. Sie können durch entsprechende mechanische Translations-, und Koppelungsmittel realisiert werden. Statische (« eingefrorene ») Verkippungs-Varianten zeigen beispielsweise die Figuren 4-6, 10 und 11.

Die Fig. 2 zeigt eine Seitenansicht (Längsschnitt) einer zweiten Ausführungsform der vorliegenden Erfindung. Das vertikale Rahmenstativ 1 ist « geschlossen » ; das vordere Stativteil 3 und das hintere Stativteil 4 sind mit einem Rahmenstativ-Basisteil und einer Rahmenstativ-Deckplatte verbunden. Der Rechteck-Rahmen ist ganzstückig ausgeführt. Der Bereich der Fußplatte 46 ist zum Beobachter hin verlängert und trägt das Bedienpult 45. Eine Laminar-Flow-Objektspüleinrichtung ist im Bereich der Deckenplatte durch die Luftdurchtrittsöffnungen 48 und im Bereich des Fußplatten-Oberteils durch die Luftabsaugeöffnungen 49 — jeweils wieder in Matrixanordnung — sowie durch den Schlauch 50 gekennzeichnet. Das Vertikal-Rahmenstativ 1 enthält im Bereich seiner vertikalen Stativteile 3 und 4, und zwar jeweils an deren dem Objekt 5 abgewandten Seiten, Anflanschflächen 31 für das Ansetzen externer Module, beispielsweise eines Lampenhauses 26 oder eines optische Bauelemente 17, 18 enthaltenden Bauteils, auf welchen ein Binokulartubus, der beispielsweise einen Fotostutzen 16 aufweisen kann, aufsetzbar ist.

Der optische Strahlenverlauf ist in Fig. 2 als Durchlicht-Beleuchtungsstrahlengang dargestellt. Von einer Lichtquelle 27 im Lampenhaus 26 gelangt der Auflicht-Beleuchtungsstrahl 29 über einen Klappspiegel 25 als geknickter Strahlengang zum als Kondensor wirkenden Objektiv 23, nachdem er zunächst eine Auflichtbeleuchtungsoptik 28, sodann ein Umlenkelement 30, das ein drehbar gelagertes Umlenkprisma sein kann, jedoch im vorliegenden Fall als (zweiter) Klappspiegel dargestellt ist, danach ein weiteres Umlenkelement 32, anschließend eine zweite Auflichtbeleuchtungsoptik 33, sodann eine Interferenzkontrast-Einrichtung (Polarisator) 34, weiterhin eine λ-Platte, nachfolgend ein Autofokussystem und diesem nachgeordnete Blenden und schließlich ein mit einer teildurchlässigen Strahlenteilungsfläche 20 ausgestattetes Umlenkelement passiert hat, und von dort aus auf das Auflichtobjekt, beispielsweise einen Wafer. Das vom Objekt ausgehende Abbildungs- und/oder Meßstrahlenbündel gelangt durch das Objektiv 33 und nach Durchtritt durch die Strahlenteilungsfläche 20 und einen Analysator 21 auf einen Strahlenteilungswürfel 19, von dem aus ein Meß-Teilstrahlenbündel abgezweigt wird, welches nach Passieren einer Tubuslinse 38, einer weiteren Optik 39, eines wahlweise einschaltbaren Längen- bzw. Winkelmeßsystems 40 und einer weiteren Optik 41 zu einem beispielsweise modular angeordneten Intensitäts- bzw. Spektralmeßgerät geführt wird.

Der den Strahlenteilungswürfel 19 passierende Teil des Abbildungsstrahlenbündels wird über eine Tubuslinse 18 und ein Umlenkprisma 17 zu einem im Binotubusgehäuse vorgesehenen Umlenk-Teilerprisma 15 geführt, von wo aus das eigentliche Beobachtungs-Teilstrahlenbündel in das oder die Okulare 14, und ein Registrier-Teilstrahlenbündel in einen Fotostutzen 16 gelenkt wird.

Der Durchlicht-Beleuchtungsstrahlengang wird in der Weise realisiert, daß das von der Lichtquelle 27 ausgehende Beleuchtungsstrahlenbündel 44 bei aus dem Strahlengang ausgeschwenkter Stellung des Klappspiegels 25 zunächst eine Leuchtfeldblende 24 und sodann eine Durchlichtbeleuchtungsoptik 43 durchläuft, um anschließend ein — beispielsweise großflächiges — Durchlichtobjekt 6 zu durchdringen.

Die beschriebenen und gezeichneten Optikanordnungen sind in bekannter Weise modifizierbar. So kann beispielsweise eine kombinierte Auflicht-Durchlicht-Beleuchtung dadurch realisiert werden, daß vom Lampenhaus 26 der zuletzt beschriebene Durchlichtbeleuchtungsstrahlengang ausgeht, während von einem zweiten, nicht eingezeichneten Lampenhaus an der im Fußplattenbereich befindlichen zweiten Anflanschstelle 31 ein Auflichtbeleuchtungsstrahlengang ausgeht, wobei lediglich der zweite Klappspiegel 30 außer Wirkstellung zu bringen ist.

Der Objekttisch 5 zeigt in seinem oberen Plattenbereich in schematischer Darstellung Vakuum- bzw. Ansaug-Kanäle 54, die beispielsweise radialstrahlig und/oder konzentrisch angeordnet sein können und mit einer Vakuumerzeugungsvorrichtung über einen Vakuumschlauch 53 verbunden sind. Ein zweidimensionales, gegen mechanische Beschädigungen hochempfindliches Präzisionsobjekt 6 kann mittels der einstellbaren Saugvorrichtung schonend gehaltert werden. Zusätzlich können Anlage-Zapfen oder lineare bzw. kreissegmentartige Aufkantungen oder Spannelemente als Positionier-, Halte- oder Justierhilfen auf der Objekttischoberfläche vorgesehen sein.

Der An- und Abtransport des Objektes, insbesondere eines zerbrechlichen großformatigen Wafers, der sich in Vertikallage bzw. in leichter Schräg-Rückenlage befindet, gelingt in objektschonender, kontaminationsungefährdeter, für den Blick des Beobachters frei zugänglicher, apparativ-rationeller Weise. Der drehbare, vgl. den Kreispfeil 52, Objekttisch 5 kann zur Herstellung des Fokuszustandes außerdem in Richtung des Doppelpfeiles 51 achsial verstellbar sein. Es ist ebenso möglich, den Objektivrevolver 22 mit einer Achsialverschiebungsvorrichtung auszustatten. Diese Translationen können vorteilhafterweise von einem inkorporierten Autofokussystem 36 automatisch ausgeführt werden. Vor allem wird die Objektivrevolverdrehung motorisch vollzogen, um keinerlei Manipulationen in Objektnähe durchführen zu müssen, die zwangsläufig zu einer « Mikroverschmutzung » des kompliziert strukturierten integrierten Schaltkreises (Objekt) führen würden.

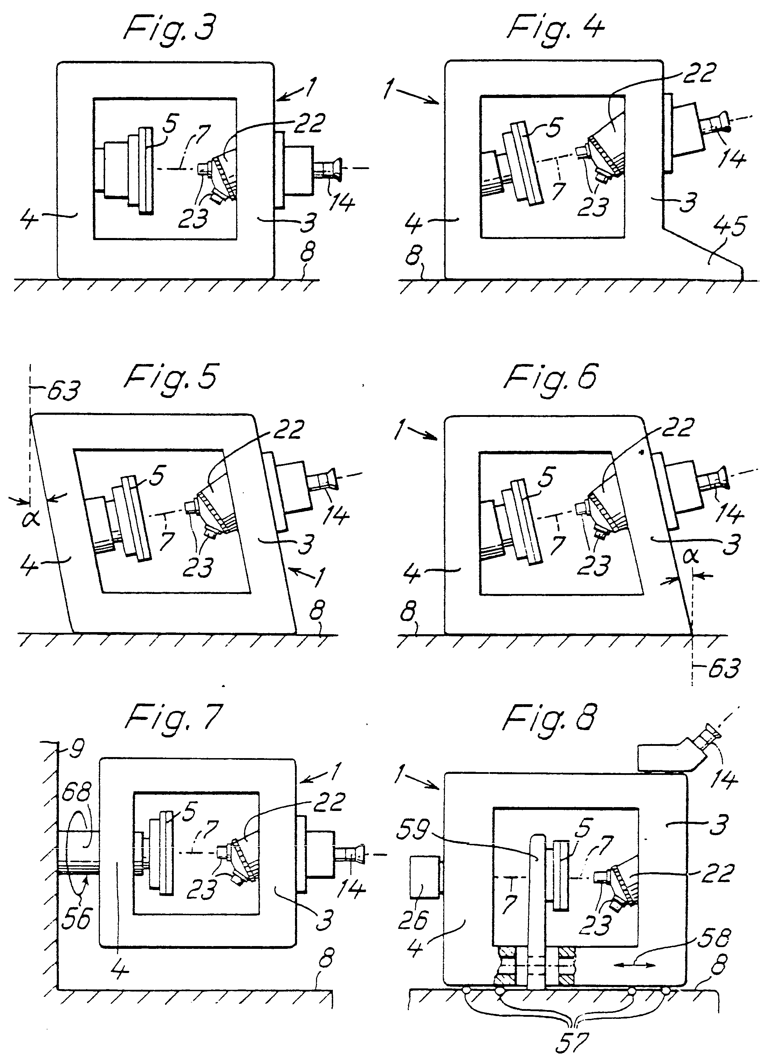

Die Figuren 3-6 zeigen weitere Varianten von geschlossenen Vertikal-Rahmenstativ-Konstruktionen in stark schematisierter Darstellung, die hinsichtlich ihrer optischen Bauglieder-Bestückung prinzipiell analog zu den in Figur 2 erläuterten Gegebenheiten ausgerüstet sein können, insbesondere also auch kombinierte Auflicht/Durchlicht-Beleuchtungsstrahlengänge aufweisen können. Außerdem können sie prinzipiell Anflansch- und Aufsatz-Schnittstellen aufweisen. Hinsichtlich ihrer ergonomischen Konstruktionsmerkmale (senkrechte Bedienleisten ; vorgezogene Bedienpulte ; Laminar-Flow-Einrichtungen) gilt ebenfalls das zu den Figuren 1a-2 bereits ausgeführte.

Fig. 3 zeigt einen quadratischen Rahmenstativ-Längsschnitt mit vertikaler Objekttischebene und horizontaler optischer Achse 7.

Die Darstellung in Fig. 4 unterscheidet sich von derjenigen in Fig. 3 lediglich dadurch, daß der Objekttisch 5 eine festgelegte Schrägrückenlage aufweist und die optische Achse in entsprechender Weise aus ihrer Horizontallage ausgelenkt ist. Eine derartige Schräglage des Objektes (nicht mit dargestellt) auf dem Objekttisch 5 ist in den Fällen zweckmäßig, in denen auf eine permanente Vakuumansaugung des Flächenobjektes verzichtet wird bzw. dann, wenn ein Objekt, beispielsweise eine Rasterplatte mit einer Vielzahl von Öffnungen oder ein netzstrukturiertes bzw. ein mit durchgängigen Poren versehenes Flächenobjekt zu untersuchen ist. An sich bekannte Anlageleisten oder -Noppen oder flächige Ausnehmungen im Unterbereich des Objekttisches gewährleisten eine stabile Ruhelage des Objektes.

In Fig. 5 ist ein parallelogrammförmiges Vertikal-Rahmenstativ 1 dargestellt. Die Schräglage der beiden Stativteile 3 und 4 — gekennzeichnet durch den Winkel α zwischen der eingezeichneten Vertikalebenen-Spur 63 und der Fläche des hinteren Stativteils 4 — entspricht derjenigen des Objekttisches 5.

Fig. 6 zeigt einen trapezförmigen Rahmen-Längsschnitt mit ergonomisch vorteilhafter Schräglage des vorderen Stativteils. Wenngleich aus Vereinfachungsgründen bislang immer von einem Vertikal-Rahmenstativ gesprochen wurde, so ist doch darauf hinzuweisen, daß das Gesamt-Gerät eine solche Rahmenbreite aufweisen muß, daß ein kompaktes, gedrungenes, äußere Störeinflüsse abschirmendes, stabiles, mit allen bekannten mikroskopischen Optikbauteilen bestücktes Gehäuse resultiert.

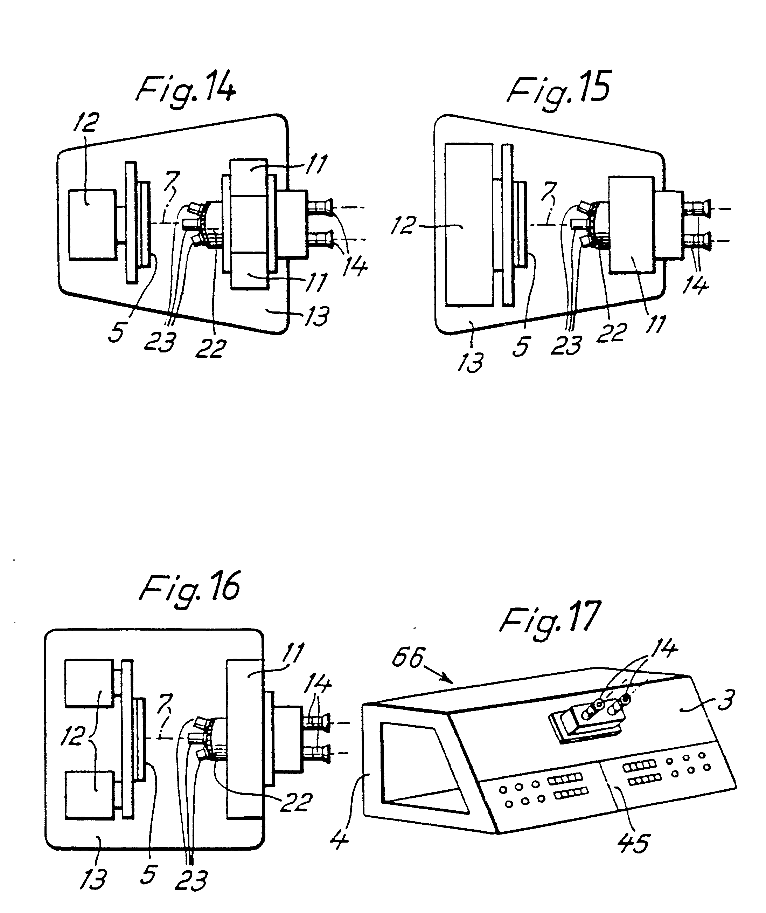

In Fig. 17 ist eine aus Fig. 6 ableitbare Hohlprofil-Konstruktion 66 dargestellt. Die Bedienelemente finden sich ausnahmslos an oder in dem vorn unten angeordneten Bedienpult 45. Schottet man beide Seiten des Gehäuse-Querkanals ab, so kann bei kontrollierter Luftzu- und -abführung über die bereits erwähnten Öffnungen 48 und 49 ein abgeschlossenes Flow-Innenströmungssystem oder aber eine gezielte Schutzgasatmosphäre erzeugt werden. Vor allem aber kann das sich quer zur optischen Achse des Gerätes erstreckende rohrförmige Tunnelgehäuse 66 beim An- und Abtransport der zu untersuchenden Wafer eine besondere Schutzfunktion übernehmen. Alle anderen offenbarten Rahmen-Längsschnittgeometrien können prinzipiell auch als Hohlprofil-Tunnel-Querschnitte dienen. Dies gilt auch für die weiter unten zu erörternden Rahmenstativ-Konstruktionen in offener Bauweise.

In Fig. 7 ist eine Spezialvariante dargestellt. Ein geschlossenes Rahmenstativ 1 wird im Bereich seines hinteren Stativteils 4 von einer zylinderförmigen Objekttischhalterung 68 durchsetzt, die an einer Vertikal-Anschlagfläche 9 — beispielsweise an einer Wand — entweder ortsfest oder rotatorisch gehaltert ist, vgl. den Drehpfeil 56. Die optische Achse 7 des Mikroskops verläuft waagerecht und steht auf der vertikalen Objekttischebene senkrecht. Bei entsprechender Lagerung, Kopplung bzw. Entkopplung der relativ zueinander bewegbaren Geräteteile 68 und/oder 5 und/oder 1 kann bei feststehendem Objekttisch 5 beispielsweise eine Rotation bzw. eine schrittweise — etwa jeweils um 60° erfolgende — Drehung um die Achse 7 erfolgen. Andererseits ist es auch möglich, das Rahmenstativ 1 zusammen mit dem Objekttisch 5 in entsprechender Weise zu drehen. Diese Möglichkeiten sind vor allem dann außerordentlich bedeutsam, wenn das in Fig. 7 dargestellte Gesamtgerät etwa in der Weise aufgestellt und eingesetzt wird, daß nur die Anschlagfläche 9 vorhanden ist und das Gerät als zentrale Inspizier- und Meßstation von sternförmig angeordneten Transportzuführungs- und Transportabführungs-Straßen umgeben ist, welche in einer zur Fläche 9 parallelen Prozeßstraßenebene angeordnet sind. Durch schrittweises — etwa mikroprozessorgesteuertes — Verdrehen des Rahmenstativs 1 kann eine vorgebbare Weichenstellung für die Annahme bzw. die Abgabe von Serien-Untersuchungsobjekten, vor allem von Wafern, erzielt werden. Dabei ist immer eine erschütterungsfreie, robuste Aufhängung der für ein mikroskopisches Gerät erforderlichen Optikbaugruppen gewährleistet.

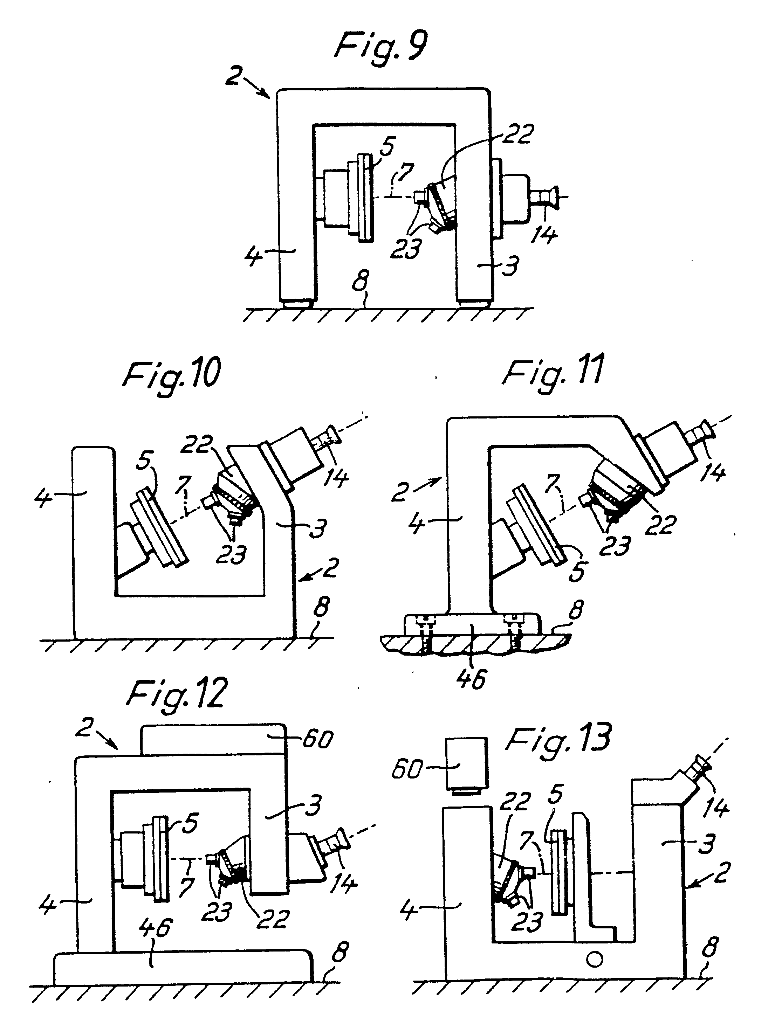

In Fig. 8 ist eine andere Form der Relativverstellung des vertikalen Objekttisches 5 zum Rahmenstativ 1 gezeigt. Das Rahmenstativ 1 wird mit seinem Basisrahmenteil auf der Horizontalauflagefläche 8 kugellagergeführt, vgl. die Bezugsziffer 57, während die Halterung für den Objekttisch 5 ortsfest auf der Fläche 8 befestigt ist. Es ist indes ebenfalls möglich, den Objekttischhalter achsialverschieblich zu lagern.

In den Fig. 9 bis 13 sind « offene » Vertikal-Rahmenstativ-Konstruktionen unterschiedlicher Rahmen-Längsschnittgeometrie, unterschiedlicher Lage der jeweiligen Objekttischebene sowie unterschiedlicher Lage der Rahmen-« Öffnung » dargestellt. Auch hier gilt bezüglich der Anordnung der optischen Bauglieder bzw. der Adaptionsmöglichkeiten von Modulen bzw. der Orientierung der optischen Achse 7 und damit des Objekttisches 5 sowie bezüglich der Luftströmungs-Verhältnisse prinzipiell das weiter oben bei der Erläuterung der « geschlossenen » Rahmenstativ-Varianten Gesagte. In Fig. 9 ist eine Konstruktion mit zur Horizontal-Auflagefläche 8 weisender Rahmenöffnung angegeben. In Deckenbereich dieses Stativs können wiederum Luftdurchtrittsöffnungen 48 bzw. Luftabsaugeöffnungen 49 vorgesehen sein. Auch können dort zusätzlich nicht mit dargestellte Lichtquellen für eine Schrägauflichtbeleuchtung des jeweiligen Objektes vorhanden sein. Darüber hinaus ist es auch möglich, den Objekttisch 5 — wie etwa in Fig. 8 gezeigt — ortsfest auf der Horizontal-Auflagefläche 8 zu halten und das mobile Gerät 2 über den vertikal gehalterten Objekttisch 5 zu « stülpen ». Bei einer extremen Verlängerung des Rahmenstativs 2 quer zur optischen Achse 7 nach Art des in Fig. 17 dargestellten und einer hintereinander angeordneten Fließbandkette mit einer Vielzahl von Vertikal-Objekthalterungen, die der Reihe nach in Mikroskopierposition gefahren werden, kann die dargestellte Ausführungsform gleichsam als Inspektions-Terminal innerhalb einer geschlossenen Wafer-Transport-Straße dienen.

Schließlich sind in den Figuren 14 bis 16 stark schematisierte Draufsichten von vertikalen Stativ- bzw. Einzelwand-Kombinationen gezeigt. In Fig. 14 besteht die Einrichtung aus zwei vorderen Einzelstativteilen 11, die gemeinsam das eigentliche aus einer Okular- und einer Objektiveinheit bestehende Beobachtungssystem tragen. In Fig. 15 ist der hintere Stativteil 12 als Vertikal-Wand verbreitert. Alternative Ausbildungsformen sind Portal, Rundbügel, Winkelbügel und dergleichen. Natürlich könnten auch mehr als zwei vordere bzw. hintere Einzelstativteile 11 bzw. 12 vorgesehen und mittels einer Strativfußplatte 13 und/oder einer nicht mit dargestellten Stativdeckplatte versehen sein. Zusätzlich ergibt sich die Möglichkeit, mehr als ein Binokular-Gehäuse — etwa nach Art einer Multi-Diskussionsbrücke — oder mehr als einen Objekttisch — etwa nach Art eines Vergleichsmikroskops — vorzusehen.

Mit den beschriebenen erfindungsgemäßen Ausführungsformen wird ein optisches Beobachtungsgerät angegeben, mit dem ein kontaminationsfreies Handling, Inspizieren und Ausmessen von hochpräzisen, beschädigungsempfindliche Objekten, insbesondere großflächigen Wafern, auf optimale Weise ermöglicht wird. Der Geräteaufbau ist stabil, es sind vergrößerte Kreuztischverfahrbereiche möglich, dem Beobachter wird ein ergonomisches System mit übersichtlichen, den empfindlichen Objektbereich nicht tangierenden Bedienmöglichkeiten an die Hand gegeben; die Einrichtung ist ausbaufähig für alle in der Mikroskopie bekannten Spezialuntersuchungs- und Meßtechniken; die Automatisierung und Fernsteuerung aller Manipulierfunktionen ist realisierbar, gleichwohl ist auch eine volle Funktionsfähigkeit unter Beibehaltung aller genannten Vorteile bei manuellem Betrieb möglich; Objekte beliebiger Flächenausdehnung sind für eine Untersuchung geeignet; Laminar-Flow im Objektbereich sorgt für Staubfreiheit; das Gesamtgerät hat einen geringen Grundflächenbedarf.

**Patentansprüche**

1. Durchlicht- und/oder Auflichtmikroskop, das folgende Baumerkmale aufweist:

a) das Mikroskopgehäuse besteht aus einem Rahmenstativ (1, 2; 67) in offener (Fig. 1) oder geschlossener Bauweise (Fig. 2) mit einem — aus der Sicht des Beobachters (47) — vorderen (3) und einem hinteren (4) Stativ-Teil und einer Stativgrundplatte (46, 13) und bzw. oder einer die beiden Stativ-Teile (3, 4) verbindenden Stativdeckplatte;

b) zwischen dem vorderen und dem hinteren Stativ-Teil (3, 4) befindet sich der im wesentlichen vertikal angeordnete Objekttisch (5);

c) das vordere Stativ-Teil (3) trägt den Beobachtungsteil (14, 22) des Mikroskops, dessen Achse (7) mit der Horizontalen einen zwischen 0° und 30° liegenden, vorzugsweise jedoch 0° betragenden Winkel α bildet;

d) im Oberbereich der Fußplatte (46) und/oder im Unterbereich einer Stativdeckplatte und/oder in den jeweils gegenüberliegenden Stativ-Teilen (3, 4) sind eine Vielzahl von Luftöffnungen (48, 49) angeordnet, welche mit einer Raumluft-Durchsaugevorrichtung (50) in Verbindung stehen, so daß das Objekt (6) einer gezielt einstellbaren laminaren Luft- bzw. Schutzgasströmung (48-50) ausgesetzt ist.

2. Mikroskop nach Anspruch 1, dadurch gekennzeichnet, daß die Stativteilen (11, 12) als Stativsäulen ausgebildet sind.

3. Mikroskop nach mindestens einem der Ansprüche 1 und 2, dadurch gekennzeichnet, daß mindestens zwei vordere (11) und/oder mindestens zwei hintere (12) Stativsäulen vorgesehen sind (Fig. 14 bis 16).

4. Mikroskop nach Anspruch 1, dadurch gekennzeichnet, daß die Stativteile (11, 12) als Stativwand bzw. als Stativwände bzw. als Portal (e) bzw. als Rund- oder Winkelbügel ausgebildet sind.

5. Mikroskop nach mindestens einem der vorhergehenden Ansprüche, dadurch gekennzeichnet, daß die Stativteile (11, 12) bzw. die Stativ-Teile (3, 4) mit der Vertikalen (63) einen zwischen 0° und 30° liegenden, vorzugsweise jedoch 0° betragenden Winkel α bilden.

6. Mikroskop nach mindestens einem der vorhergehenden Ansprüche, dadurch gekennzeichnet, daß in den Stativteilen (11, 12), der Stativfußplatte (13 bzw. 46) und — gegebenenfalls — der Stativdeckplatte bzw. in dem Rahmenstativ (1, 2; 67) die optischen Elemente (19-21, 23-25, 28, 30, 32-39, 41 und 43) zur Strahlenführung und Strahlenbeeinflussung untergebracht sind (Fig. 2).

7. Mikroskop nach mindestens einem der vorhergehenden Ansprüche, dadurch gekennzeichnet, daß alle optischen, mechanischen und elektromechanischen Bedienelemente im beobachterseitigen Teil der Stativfußplatte (13) und/oder am vorderen Stativteil (11) bzw. am vorderen Rahmenteil (45) angeordnet sind.

8. Mikroskop nach mindestens einem der vorhergehenden Ansprüche, dadurch gekennzeichnet, daß der vordere Stativteil (11) bzw. das vordere Stativ-Teil (3) Aufnahmen zum auswechselbaren Einschieben von in der Mikroskopiertechnik gebräuchlichen Beleuchtungs-, Beobachtungs-, Meß- bzw. Registrier-Modulen (34, 36, 42) aufweist (aufweisen).

9. Mikroskop nach mindestens einem der vorhergehenden Ansprüche, dadurch gekennzeichnet, daß das hintere Stativ-Teil (4) bzw. die hintere(n) Stativsäule(n) (12) — vorzugsweise in ihrem Stativfußplattenbereich — Mittel (31) zum Ansetzen mindestens eines externen Lampenhauses (26) aufweist (aufweisen).

10. Mikroskop nach mindestens einem der vorhergehenden Ansprüche, dadurch gekennzeichnet, daß der Objekttisch (5) als Drehkreuztisch ausgebildet ist und Anschlag- und/oder Haltemittel zum Positionieren von Objekten (6) — vorzugsweise von großflächigen, kontaminationsgefährdeten Präzisionsobjekten — aufweist.

11. Mikroskop nach mindestens einem der vorhergehenden Ansprüche, dadurch gekennzeichnet, daß der Objekttisch (5) magnetische oder pneumatische Haft- bzw. Ansaugvorrichtungen (53, 54) aufweist.

12. Mikroskop nach mindestens einem der vorhergehenden Ansprüche, dadurch gekennzeichnet, daß der Objekttisch (5) an dem hinteren Stativ-Teil (4) bzw. dem hinteren Stativteil (12) gehalten ist.

13. Mikroskop nach mindestens einem der vorhergehenden Ansprüche, dadurch gekennzeichnet, daß der Objekttisch (5) auf der Stativgrundplatte (46) bzw. auf dem entsprechenden Stativ-Teil — vorzugsweise achsialverschieblich — gehalten ist (Fig. 13).

14. Mikroskop nach mindestens einem der Ansprüche 1 bis 11, dadurch gekennzeichnet, daß der Objekttisch (5) mit einer horizontalen Mikroskopauflage (8) starr verbunden und das Rahmenstativ (1, 2) relativ zu Objekttisch (5) achsialverschieblich gelagert ist (Fig. 8).

15. Mikroskop nach mindestens einem der Ansprüche 1 bis 12, dadurch gekennzeichnet, daß das Rahmenstativ (1) um die horizontal verlaufende optische Achse (7) des Mikroskops rotatorisch gelagert ist (Fig. 7).

16. Mikroskop nach Anspruch 15, dadurch gekennzeichnet, daß der an einer Vertikalwand (9) gehalterte Objekttisch (5) der Rotationsbewegung (56) des Rahmenstativs (1) folgt (Fig. 7).

17. Mikroskop nach den Ansprüchen 15 und 16, dadurch gekennzeichnet, daß der achsialverschiebliche Objekttisch (5) rotationsinvariant gehaltert ist (Fig. 7).

18. Mikroskop nach mindestens einem der vorhergehenden Ansprüche, dadurch gekennzeichnet, daß das Rahmenstativ (1, 2) einen rechteckigen oder quadratischen Längsschnitt aufweist und die Fußplatte (46) beobachterseitig verlängert und als manuelles Bedienpult (45) ausgebildet ist (Fig. 1, 2 bzw. 4).

19. Mikroskop nach mindestens einem der vorhergehenden Ansprüche, dadurch gekennzeichnet, daß das Rahmenstativ (1, 2) einen trapez- oder parallelogrammförmigen Längsschnitt mit zum Beobachter (47) weisenden, schrägen Rahmenvorderteil (3) aufweist (Fig. 5, 6, 10 und 11).

20. Mikroskop nach Anspruch 19, dadurch gekennzeichnet, daß das beobachterseitige Stativ-Teil (3) und der Objekttisch (5) in zueinander parallelen Ebenen angeordnet sind.

21. Mikroskop nach mindestens einem der vorhergehenden Ansprüche, dadurch gekennzeichnet, daß das Gehäuse ein — zumindest teilweise doppelwandiger — Hohlprofilkörper (66) mit quer zur optischen Achse verlaufender Längserstreckung nach Maßgabe der jeweiligen Rahmen-längsschnittgeometrie ist (Fig. 17).

22. Mikroskop nach mindestens einem der vorhergehenden Ansprüche, dadurch gekennzeichnet, daß an bzw. auf dem — vorzugsweise vorderen — Stativ- bzw. Rahmenteil Mittel (31) zum Aufsetzen und Ankoppeln spezieller Mikroskop-Module (14-16; 17, 18; 22; 26; 31; 60; 61) vorgesehen sind.

23. Mikroskop nach mindestens einem der vorhergehenden Ansprüche, dadurch gekennzeichnet, daß es sich in einem atmosphärisch abgeschlossenen System befindet und mit mechanischen und/oder optischen und/oder elektronischen Mitteln zum Fernbedienen der Manipulier- und Bedienfunktionen (45) ausgestattet ist (Fig. 17).

24. Verwendung eines Mikroskops nach mindestens einem der vorhergehenden Ansprüche als Wafer-Inspektions- und Meßeinrichtung.

25. Verwendung eines Mikroskops nach mindestens einem der vorhergehenden Ansprüche als Strukturbreiten- bzw. Schichtdickenmeßeinrichtung.

26. Verwendung eines Mikroskops nach mindestens einem der vorhergehenden Ansprüche als integriertes Gerät innerhalb einer Wafer-Fertigungs- und Kontroll-Straße.

## Claims

1. Transmitted and/or incident light microscope which displays the following constructional features :

a) the microscope housing consists of a frame stand (1, 2 ; 67) in open (Fig. 1) or closed (Fig. 2) mode of construction with — from the view of the observer (47) — a front (3) and a rear (4) stand part and a stand base plate (46, 13) and/or a stand cover plate connecting both the stand parts (3, 4) ;

b) the substantially vertically arranged object table (5) is disposed between the front and the rear stand part (3, 4) ;

c) the front stand part (3) carries the observation part (14, 22) of the microscope, the axis (7) of which forms an angle α lying between 0° and 30°, preferably however amounting to 0°, with the horizontal ;

d) a plurality of air openings (48, 49), which stand in communication with a device (50) for sucking room air through, are arranged in the upper region of the pedestal plate (46) and/or the lower region of a stand cover plate and/or the stand parts (3, 4) respectively lying opposite so that the object (6) is exposed to a laminar air or protective gas current (48 to 50), which is adjustable in targeted manner.

2. Microscope according to claim 1, characterised thereby, that the stand parts (11, 12) are constructed as stand columns.

3. Microscope according to at least one of the claims 1 and 2, characterised thereby, that at least two front (11) and/or at least two rear (12) stand columns are provided (Figs. 14 to 16).

4. Microscope according to claim 1, characterised thereby, that the stand parts (11, 12) are constructed either as stand wall or as stand walls or as portal(s) or as round or angled brackets.

5. Microscope according to at least one of the preceding claims, characterised thereby, that either the stand parts (11, 12) or the stand parts (3, 4) form an angle α lying between 0° and 30°, preferably however amounting to 0°, with the vertical (63).

6. Microscope according to at least one of the preceding claims, characterised thereby, that the optical elements (19 to 21, 23 to 25, 28, 30, 32 to 39, 41 and 43) for the ray guidance and ray influencing are housed in the stand parts (11, 12), the stand pedestal plate (13 or 46) and, in a given case, either in the stand cover plate or in the frame stand (1, 2 ; 67) (Fig. 2).

7. Microscope according to at least one of the preceding claims, characterised thereby, that all optical, mechanical and electromechanical operating elements are arranged at that part of the stand pedestal plate (13), which faces the observer, and/or either at the front stand part (11) or at the front frame part (45).

8. Microscope according to at least one of the preceding claims, characterised thereby, that either the front stand part (11) or the front stand part (3) displays receptacles for the exchangeable insertion of illumination, observation, measurement or recording modules (34, 36, 42) customary in the microscopy technique.

9. Microscope according to at least one of the preceding claims, characterised thereby, that either the rear stand part (4) or the rear stand column(s) (12) — preferably in their stand pedestal plate region — display(s) means (31) for mounting at least one external lamp housing (26).

10. Microscope according to at least one of the preceding claims, characterised thereby, that the object table (5) is constructed as rotary stage and displays abutment and/or retaining means for the positioning of objects (6), preferably of precision objects of large area and endangered by contamination.

11. Microscope according to at least one of the preceding claims, characterised thereby, that the object table (5) displays magnetic or pneumatic adhesion or suction devices (53, 54).

12. Microscope according to at least one of the preceding claims, characterised thereby, that the object table (5) is retained either at the rear stand part (4) or the rear stand part (12).

13. Microscope according to at least one of the preceding claims, characterised thereby, that the object table (5) is retained either on the stand base plate (46) or on the corresponding stand part, preferably to axially displaceable (Fig. 13).

14. Microscope according to at least one of the claims 1 to 11, characterised thereby, that the object table (5) is rigidly connected with an horizontal microscope support (8) and the frame stand (1, 2) is borne to be axially displaceable relative to the object table (5) (Fig. 8).

15. Microscope according to one of the claims 1 to 12, characterised thereby, that the frame stand (1) is borne to be rotatable about the horizontally extending optical axis (7) of the microscope (Fig. 7).

16. Microscope according to claim 15, characterised thereby, that the object table (5) is retained at a vertical wall (9) and follows the rotary movement (56) of the frame stand (1) (Fig. 7).

17. Microscope according to the claims 15 and 16, characterised thereby, that the axially displaceable object table (5) is retained to be rotationally invariable (Fig. 7).

18. Microscope according to at least one of the preceding claims, characterised thereby, that the frame stand (1, 2) displays a rectangular or square longitudinal section and the pedestal plate (46) is prolonged at the observer end and constructed as manual operating desk (45) (Figs. 1, 2 or 4).

19. Microscope according to at least one of the preceding claims, characterised thereby, that the frame stand (1, 2) displays a longitudinal section in the shape of a trapezium or parallelogram with an inclined front frame part (3) pointing to the observer (47) (Figs. 5, 6, 10 and 11).

20. Microscope according to claim 19, characterised thereby, that the stand part (3), which faces the observer and the object table (5) are arranged in mutually parallel planes.

21. Microscope according to at least one of the preceding claims, characterised thereby, that the housing is a hollow profile body (66), which at least in part is double walled, with a longitudinal extent, in a course transverse to the optical axis, corresponding to the respective geometry of the longitudinal frame section (Fig. 17).

22. Microscope according to at least one of the preceding claims, characterised thereby, that means (31) for the mounting and coupling of special microscope modules (14 to 16 ; 17, 18 ; 22 ; 26 ; 31 ; 60 ; 61) are provided at or on the preferably frontal stand or frame part.

23. Microscope according to at least one of the preceding claims, characterised thereby, that it is disposed in an atmospherically closed system and equipped with mechanical and/or optical and/or electronic means for the remote control of the manipulating and operating functions (45) (Fig. 17).

24. Use of a microscope according to at least one of the preceding claims as wafer inspection and measuring equipment.

25. Use of a microscope according to at least one of the preceding claims as structure width or layer thickness measuring equipment.

26. Use of a microscope according to at least one of the preceding claims as integrated device within a wafer production and inspection line.

**Revendications**

1. Microscope à éclairage par transmission et/ou par lumière incidente qui présente les caractéristiques de construction suivantes :

    (a) le carter de microscope se compose d'un statif à bâtir (1, 2 ; 67) à mode de construction ouvert (Fig. 1) ou fermé (Fig. 2) avec — à la vue de l'observateur (47) — un élément de statif avant (3) et un élément de statif arrière (4) et une plaque de base de statif (46, 13) et/ou une plaque de recouvrement de statif reliant les deux éléments de statif (3, 4) ;

(b) entre les éléments de statif avant et arrière (3, 4) se trouve la platine porte-objet (5) disposée essentiellement verticalement ;

(c) l'élément de statif avant (3) porte l'élément d'observation (14, 22) du microscope dont l'axe (7) forme un angle $\alpha$ compris entre 0° et 30°, mais de préférence égal à 0° ;

(d) dans la partie supérieure de la plaque de pied (46) et/ou dans la zone inférieure d'une plaque de recouvrement de statif et/ou dans les éléments de statif (3, 4) respectivement opposés est disposée une multiplicité d'orifices d'air (48, 49), lesquels sont en liaison avec un dispositif d'aspiration traversante d'air ambiant (50), de sorte que l'objet (6) est exposé à un écoulement laminaire réglable visé d'air ou de gaz protecteur (48-50). ,

2. Microscope selon la revendication 1, caractérisé en ce que les éléments de statif (11, 12) sont réalisés sous forme de colonnes de statif.

3. Microscope selon au moins l'une des revendications 1 et 2, caractérisé en ce qu'au moins deux colonnes de statif avant (11) et/ou au moins deux colonnes de statif arrière (12) sont prévues (Fig. 14 à 16).

4. Microscope selon la revendication 1, caractérisé en ce que les éléments de statif (11, 12) sont réalisés sous forme de paroi de statif ou de parois de statif ou de portique(s) ou d'étriers ronds ou anguleux.

5. Microscope selon au moins l'une des revendications précédentes, caractérisé en ce que les éléments de statif (11, 12) ou les éléments de statif (3, 4) forment, avec la verticale (63), un angle $\alpha$ compris entre 0° et 30°, mais de préférence égal à 0°.

6. Microscope selon au moins l'une des revendications précédentes, caractérisé en ce que dans les éléments de statif (11, 12), dans la plaque de pied de statif (13 ou 46) et — éventuellement — dans la plaque de recouvrement de statif ou dans le statif à bâti (1, 2 ; 67) sont logés les éléments optiques (19-21, 23-25, 28, 30, 32-39, 41 et 43) pour le guidage des rayons et l'influencement des rayons (Fig. 2).

7. Microscope selon au moins l'une des revendications précédentes, caractérisé en ce que tous les éléments de manœuvre optiques, mécaniques et électromécaniques sont disposés dans la partie, côté observateur, de la plaque de pied de statif (13) et/ou sur l'élément de statif avant (11) ou sur l'élément de bâti avant (45).

8. Microscope selon au moins l'une des revendications précédentes, caractérisé en ce que la portion de statif avant (11) et/ou l'élément de statif avant (3) présente (présentent) des logements pour l'insertion interchangeable de modules d'éclairage, d'observation, de mesure ou d'enregistrement (34, 36, 42) usuels dans la technique de la microscopie.

9. Microscope selon au moins l'une des revendications précédentes, caractérisé en ce que l'élément de statif arrière (4) ou la ou les colonne(s) de statif arrière (12) — de préférence dans sa zone de plaque de pied de statif — comporte (comportent) des moyens (31) pour la pose d'au moins une boîte à lumière externe (26).

10. Microscope selon au moins l'une des revendications précédentes, caractérisé en ce que la platine porte-objet (5) est réalisée sous forme de platine rotative à mouvements croisés et comporte des moyens de butée et/ou de maintien pour le positionnement d'objets (6) — de préférence d'objets de précision à grande surface et à risque de contamination.

11. Microscope selon au moins l'une des revendications précédentes, caractérisé en ce que la platine porte-objet (5) comporte des dispositifs d'adhésion ou d'aspiration magnétiques ou pneumatiques (53, 54).

12. Microscope selon au moins l'une des revendications précédentes, caractérisé en ce que la platine porte-objet (5) est supportée sur l'élément de statif arrière (4) ou sur l'élément de statif arrière (12).

13. Microscope selon au moins l'une des revendications précédentes, caractérisé en ce que la platine porte-objet (5) est supportée — de préférence de façon axialement déplaçable — sur la plaque de base de statif (46) ou sur l'élément de statif correspondant (Fig. 13).

14. Microscope selon au moins l'une des revendications 1 à 11, caractérisé en ce que la platine porte-objet (5) est reliée rigidement à une surface horizontale d'assise de microscope (8) et le statif à bâti (1, 2) est monté de façon axialement déplaçable relativement à la platine porte-objet (5) (Fig. 8).

15. Microscope selon au moins l'une des revendications 1 à 12, caractérisé en ce que le statif à bâti (1) est monté de façon rotative autour de l'axe optique (7), s'étendant horizontalement, du microscope (Fig. 7).

16. Microscope selon la revendication 15, caractérisé en ce que la platine porte-objet (5), supportée sur une paroi verticale (9), suit le mouvement de rotation (56) du statif à bâti (1) (Fig. 7).

17. Microscope selon les revendications 15 et 16, caractérisé en ce que la platine porte-objet (5), axialement déplaçable, est supportée de façon à être invariante en rotation (Fig. 7).

18. Microscope selon au moins l'une des revendications précédentes, caractérisé en ce que le statif à bâti (1, 2) présente une coupe longitudinale rectangulaire ou carrée et la plaque de pied (46) est prolongée du côté de l'observateur et réalisée sous forme de pupitre de commande manuelle (45) (Fig. 1, 2 ou 4).

19. Microscope selon au moins l'une des revendications précédentes, caractérisé en ce que le statif à bâti (1, 2) présente une coupe longitudinale trapézoïdale ou en forme de parallélogramme à élément de bâti oblique (3) orienté vers l'observateur (47) (Fig. 5, 6, 10 et 11).

20. Microscope selon la revendication 19, caractérisé en ce que l'élément de bâti (3), côté observateur, et la platine porte-objet (5) sont disposés dans des plans parallèles l'un à l'autre.

21. Microscope selon au moins l'une des revendications précédentes, caractérisé en ce que le carter est un corps en profilé creux (66) — au moins partiellement à double paroi — avec étendue longitudinale s'étendant transversalement à l'axe optique en fonction de la géométrie de coupe longitudinale de bâti respective (Fig. 17).

22. Microscope selon au moins l'une des revendications précédentes, caractérisé en ce que contre ou sur l'élément de statif ou de bâti — de préférence avant — sont prévus des moyens (31) pour la pose et l'accouplement de modules de microscope spéciaux (14-16 ; 17, 18 ; 22 ; 26 ; 31 ; 60 ; 61).

23. Microscope selon au moins l'une des revendications précédentes, caractérisé en ce qu'il se trouve dans un système clos atmosphériquement et est équipé de moyens mécaniques et/ou optiques et/ou électroniques pour la télécommande des fonctions de manipulation et de manœuvre (45) (Fig. 17).

24. Utilisation d'un microscope, selon au moins l'une des revendications précédentes, comme dispositif d'inspection et de mesure de plaquettes.

25. Utilisation d'un microscope, selon au moins l'une des revendications précédentes, comme dispositif de mesure de largeur de structure ou d'épaisseur de couche.

26. Utilisation d'un microscope, selon au moins l'une des revendications précédentes, comme instrument intégré à l'intérieur d'une ligne de fabrication et de contrôle de plaquettes.

Fig.1a

Fig.1b

Fig.1c

## Fig. 2

Fig. 3

Fig. 4

Fig. 5

Fig. 6

Fig. 7

Fig. 8

3

Fig. 9

Fig. 10

Fig. 11

Fig. 12

Fig. 13

4

Fig.14

Fig.15

Fig.16

Fig.17